# EUROPEAN PATENT SPECIFICATION

(11) **EP 1 783 209 B1**
(45) Date of publication and mention of the grant of the patent: **14.04.2010**
(21) Application number: 05765432.9
(22) Date of filing: 29.06.2005
(51) Int. Cl.: C12N 5/074, C12N 5/071, C12N 1/02, G01N 33/48, C12N 15/09

(54) **SELECTIVE CULTURE METHOD AND SEPARATION METHOD FOR SMALL HEPATOCYTES WITH THE USE OF HYALURONIC ACID**
SELEKTIVES KULTIVIERUNGSVERFAHREN UND TRENNVERFAHREN FÜR KLEINE HEPATOZYTEN MIT VERWENDUNG VON HYALURONSÄURE
PROCEDE DE CULTURE SELECTIVE ET PROCEDE DE SEPARATION POUR DE PETITS HEPATOCYTES AU MOYEN DE L'ACIDE HYALURONIQUE

(30) Priority: 29.06.2004 JP 2004191477
(43) Date of publication of application: 09.05.2007
(73) Proprietor: Japan Science and Technology Agency, Kawaguchi-shi, Saitama 332-0012 (JP)
(72) Inventor: MITAKA, Toshihiro;, Sapporo-shi, Hokkaido, 0040863 (JP); KON, Junko, Cancer Res Inst., Sapporo Medical Univ, Chuo-ku, Sapporo-shi, Hokkaido 0608556 (JP)
(74) Representative: Hallybone, Huw George
(86) International application number: PCT/JP2005/011915
(87) International publication number: WO 2006/001473

(56) References cited:
- CATAPANO G ET AL: "Morphology and metabolism of hepatocytes cultured in Petri dishes on films and in non-woven fabrics of hyaluronic acid esters" BIOMATERIALS, vol. 22, no. 7, April 2001 (2001-04), pages 659-665, XP002459221 ISSN: 0142-9612
- CATAPANO G ET AL: "The effect of oxygen transport resistances on the viability and functions ofisolated rat hepatocytes" INTERNATIONAL JOURNAL OF ARTIFICIAL ORGANS, MILAN, IT, vol. 19, no. 1, 1996, pages 61-71, XP009092370 ISSN: 0391-3988
- TRANSPLANTATION (HAGERSTOWN), vol. 77, no. 12, 27 June 2004 (2004-06-27), pages 1783-1789, XP002459230 ISSN: 0041-1337
- MITAKA T ET AL: "RECONSTRUCTION OF HEPATIC ORGANOID BY RAT SMALL HEPATOCYTES AND HEPATIC NONPARENCHYMAL CELLS" HEPATOLOGY, WILLIAMS AND WILKINS, BALTIMORE, MD, US, 1999, pages 111-125, XP002941775 ISSN: 0270-9139
- TATENO C ET AL: "GROWTH AND DIFFERENTIATION IN CULTURE OF CLONOGENIC HEPATOCYTES THAT EXPRESS BOTH PHENOTYPES OF HEPATOCYTES AND BILIARY EPITHELIAL CELLS" AMERICAN JOURNAL OF PATHOLOGY, PHILADELPHIA, PA, US, vol. 149, no. 5, November 1996 (1996-11), pages 1593-1605, XP000995580 ISSN: 0002-9440
- KON J. ET AL: 'O-20 Hyaluron-san ni yoru Kogata Kansaibo no Zoshoku Kiko no Kaiseki' DAI 12 KAI KANSAIBO KENKYUKAI, THE PLACE OF LECTURE: TOKYO DAIGAKU IGAKUBU, [Online] 08 July 2005 - 09 July 2005, XP002999531 Retrieved from the Internet: <URL:http://hepato12.umin.jp/day2.html>
- KON J. ET AL: 'Kogata Kansaibo Tokuiteki Hyomen Marker no Hatsugen Kotai ni yoru Saibo Bunri' SAISEI IRYO vol. 4, 10 February 2005, page 105, XP002999532
- TAKEDA M. ET AL: 'Hyaluronan recognition mode of CD44 revealed by cross-saturation and chemical shift perturbation experiments' J.BIOL.CHEM. vol. 278, no. 44, 2003, pages 43550 - 43555, XP002999533
- ZAVAN B. ET AL: 'Extracellular matrix-enriched polymeric scaffolds as a substrate for hepatocyte cultures: in vitro and in vivo studies' BIOMATERIALS vol. 26, no. 34, 01 July 2005, pages 7038 - 7045, XP005000795

## Description

### Field of the Invention

The present invention relates to a method of isolating and selectively culturing proliferative hepatocytes (small hepatocytes) derived from mammalian including human.

### Background of the Invention

Liver and hepatocytes possess multiple functions so that they are compared to a chemical factory in the living body. For example, more than 90% of serum proteins are produced by hepatocytes, which have a detoxification function of metabolizing toxic substances that were incorporated into or produced in the living body. Thus researchers in various institutions have been attempted to culture hepatocytes, detect harmful substances (as a bio-sensor) and allow to produce substances needed for human being in vitro by using the functions of hepatocytes. In order to supply hepatocytes used for such investigations, it is necessary to isolate mature hepatocytes for every experiment, because there are currently no cell lines possessing the differentiated functions of hepatocytes. In such circumstances, the number of obtainable cells depends on the number of hepatocytes in individuals, because a method of proliferating mature hepatocytes with the differentiated functions of hepatocytes has not been established. Therefore, a continuous supply of a large number of cells having most functions of mature hepatocyte has been desired in the development of built-in type artificial livers. It has been an important issue to develop a method of cryopreserving hepatocytes, preserving them for a long time, and reusing hepatocytes from animals including human being, which has been investigated worldwide. However, there are previously only a few reports wherein cryopreserved hepatocytes had been lively adhered to the culture dishes after being thawed and had been able to maintain about 70 to 80% of hepatic functions only for a short period. Furthermore, in those reports, the thawed hepatocytes could not proliferate and died within a short period.

Hepatic dysfunction of human beings is caused by various diseases such as hepatitis, hepatic cirrhosis and liver tumors. Because artificial liver has not been yet in the clinical stage, liver transplantation is only the ultimate treatment of such disease at present. In addition, though there are numerous patients who require the liver transplantation of the liver not only in Japan but also in many countries in the world, the number of the donors for donating the organ is only 10 % of the required number. Thus, it is demanded to develop a process for producing a liver tissue *in vitro,* which is usable for liver transplantation, a process for preparing colonies of precursor cells for liver tissue usable for the process and a process for producing them *in vitro.*

On the other hand, the inventors reported a cell population in the liver tissue which exhibited similar phenotypes for makers of mature hepatocytes such as albumin, transferrin, cytokeratin (CK) 8 or CK18 and had the ultrastructural characteristics for mature hepatocyte, while the population was composed of small cells having high proliferation ability. These cells were named "small hepatocyte" (Mitaka T. et al., Hepatology, 16, 440-447 (1992), Mitaka T., Sato F, Mizuguchi T. et al., Hepatology 29, 111-135 (1999)).

The inventors also reported a method of obtaining a fraction enriched with small hepatocytes (WO 01/92481), a method of cryopreservation of small hepatocytes retaining the liver function and proliferation capacity and a method of preparing small hepatocytes suitable for such methods (WO 01/92481). The inventors further reported a colony of small hepatocytes suitable for preparing a transplantable liver tissue, a method of preparing thereof, a method of inducing a liver tissue from small hepatocytes (WO 02/088332) and a method of estimating *in vitro* the effects of drugs, particularly the effects associated with normal liver functions. However, a marker protein which is specific for the mammalian (including human) proliferative hepatocytes (small hepatocytes) has not been clearly determined, and therefore there is place left for improving an efficient method of isolating the cells.

Additionally, there are several reports on small hepatocytes and hepatic parenchymal cells having proliferation capacity (Japanese Patent No. 3211941, Laid Open Japanese Patent Application (JP-Kokai) No.2002-078481, JP-Kokai No.09-313172, JP-Kokai No. 08-112092). JP-Kokai No.2002-045087 also describes a chimeric animal having hepatocytes derived from a heterologous animal liver including human and a test method using the chimeric animal.

### Summary of the Invention

According to the present invention, a method of isolating and selectively culturing mammalian (including human) proliferative hepatocytes (small hepatocytes) are provided.

According to the present invention, a method is also provided for efficiently separating and culturing the small hepatocytes while distinguishing them from cells which lost the permanent proliferative capacity of small hepatocytes during culture (cells having at least a part of the function of mature hepatocytes). Namely, the present invention provides a method of selectively culturing the small hepatocytes.

Since specific marker proteins for the proliferative hepatocytes (small hepatocytes) of animals including human have not been determined, improvement in the efficiency of isolation and culture method has been difficult. The inventors found that the small hepatocytes specifically expressed CD44 and that hyaluronic acid, the ligand thereof was very useful for isolating and culturing the small hepatocytes.

Accordingly, the present invention is a method of culturing small hepatocytes which comprises culturing the small hepatocytes in the presence of a carrier of which surface is coated by hyaluronic acid (hyaluronic acid-coated carrier) and/or in the presence of a carrier of which major component is hyaluronic acid (hyaluronic acid-based carrier).

Furthermore the present invention provides a method of culturing small hepatocytes which comprises culturing the small hepatocytes in the presence of a carrier of which surface is attached by hyaluronic acid (hyaluronic acid-attached carrier) and/or in the presence of a carrier of which major component is hyaluronic acid (hyaluronic acid-based carrier) and separating cells which adhere to hyaluronic acid from cells which do not adhere to hyaluronic acid.

The present invention provides a method of culturing small hepatocytes, comprising the steps of
i) culturing small hepatocytes in the presence of a carrier of which surface is attached by hyaluronic acid and/or in the presence of a carrier of which major component is hyaluronic acid;
ii) in the culture obtained in step i) separating cells which adhere to hyaluronic acid from cells which do not adhere to hyaluronic acid;
iii) releasing the small hepatocytes which is obtained in step ii) and which adhere to hyaluronic acid and is from the hyaluronic acid; and,
iv) repeating the steps i) to iii) once or more for the small hepatocytes obtained in step iii).

The present invention also provides a method of isolating small hepatocytes, comprising the steps of
i) culturing cells from a mammalian liver in the presence of a carrier of which surface is attached by hyaluronic acid and/or in the presence of a carrier of which major component is hyaluronic acid;
ii) in the culture obtained in step i) separating cells which adhere to hyaluronic acid from cells which do not adhere to hyaluronic acid;
iii) releasing the cells which is obtained in step ii) and which adhere to hyaluronic acid from the hyaluronic acid;
iv) repeating the steps i) to iii) once or more for the small hepatocytes obtained in step iii); and
v) recovering the cells released from hyaluronic acid.

### Brief Description of the Drawings

Figure 1 shows the analysis of mRNA expression levels of CD44 at the time of preparation and at each indicated time after starting culture, as determined by using RT-PCR. The longitudinal axis indicates the relative intensity and the horizontal axis indicates the days of culture.
Figure 2 shows the comparison of mRNA expression levels of CD44 in small hepatocytes and mature hepatocytes, as determined by RT-PCR. SH: small hepatocytes, MH: mature hepatocytes. The longitudinal axis indicates the relative intensity.
Figure 3 shows the results of comparison of mRNA expression levels of CD44 in small hepatocytes and mature hepatocytes, as determined by Northern blot.
SH: small hepatocytes, MH: mature hepatocytes. The longitudinal axis indicates the relative intensity. Upper represents the longer mRNA among detected mRNAs and Lower represents the smaller mRNA among detected mRNAs in the small hepatocytes.
Figure 4 shows the CD44 expression in small hepatocytes which had been induced to maturation by Matrigel, as determined by Northern Blot. SH: small hepatocytes, MH: mature hepatocytes. The longitudinal axis indicates the relative intensity.
Figure 5 shows the time course of the proliferation of small hepatocyte colonies on culture dishes coated with different amounts of hyaluronic acid. The longitudinal axis indicates area x 10⁻³ represented in mm². Positive control: culture dish for adherent cells; Negative control: culture dish for non-adherent cells; Collagen: collagen coated culture dish; 1 mg HA, 5mg HA and 10mg HA: culture dishes treated with 1 mg, 5 mg and 10 mg of hyaluronic acid, respectively.

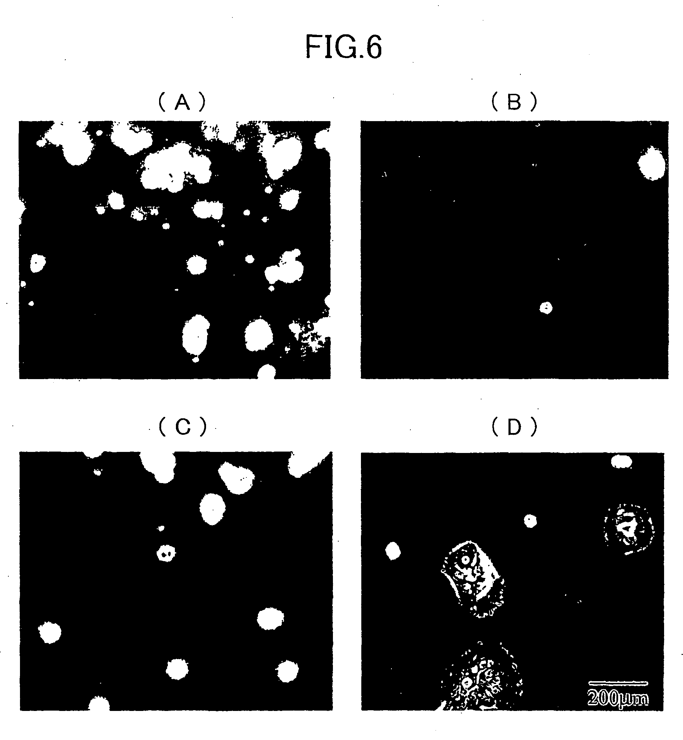

Figure 6 represents phase contrast micrographs of mature hepatocytes and small hepatocytes on culture dishes coated with hyaluronic acid. A: mature hepatocytes, day 1 of culture; B: mature hepatocytes, day 7 of culture; C: small hepatocyte, day 1 of culture, small hepatocyte day 7 of culture. Mature hepatocytes (MH) and small hepatocytes (SH) were plated at a concentration of 3 x 10⁵ cells on 60mm culture dishes coated with hyaluronic acid, respectively. The micrographs show the growth of the cells at day 1 and day 7, respectively. Small hepatocytes proliferated to form colonies, while mature hepatocytes dissociated with forming a round shape and almost all the adherent cells disappeared at day 7. The bar in the figure indicates a length of 200 micrometer.

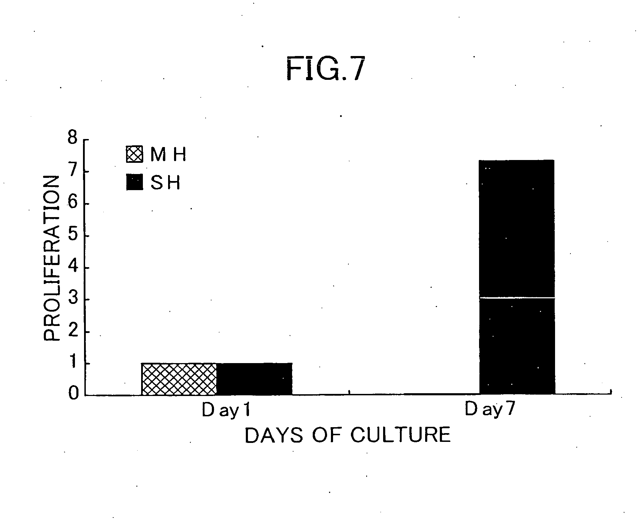

Figure 7 is a graph showing the growth of mature hepatocytes and small hepatocytes on culture dishes coated with hyaluronic acid. The survived adherent cells were counted and the numbers of cells at day 7 were relatively indicated as designating the number of cells at day 1 as one.

### Description of the Preferred Embodiments

According to the present invention, a method of efficiently isolating and culturing small hepatocytes is provided. The term "small hepatocyte" as used herein does not merely mean a small cell which is derived from a liver, but it means a small cell which may be isolated according to the following procedures or the similar procedures and which exhibits substantially similar phenotypes to a mature hepatocyte regarding the markers such as albumin, transferrin, cytokeratin (CK) 8 and CK18 and has the ultrastructural features as a hepatocyte. These cells were found by the inventors, the details of which were described in Mitaka, T. et al., Hepatology, 16, 440-447 (1992) and Mitaka, T., Sato F, Mizuguchi T. et al., Hepatology 29, 111-135 (1999).

In the present inventions, the cell fraction prepared from a liver may be directly cultured and subjected to isolation under hyaluronic acid, or a fraction enriched with small hepatocytes can be prepared by a convenient procedure and subsequently the method according to the present invention may be applied on the fraction.

A fraction enriched with the small hepatocytes may be prepared as follows. Liver-derived cells can be obtained by treating liver tissues from human being or other animals with a solution containing a collagenase and the like, such as a method similar as the method of Seglen (Seglen, PO., Methods Cell Biol., 1976, 13, 29-83). For this purpose, a conventional liver perfusion method may be used. The obtained cell suspension may be passed through a mesh of an appropriate size to remove tissue debris such as undigested tissue residues and the like, if necessary. Although the cell suspension can be directly used to separate the small hepatocytes according to the method of the present invention, it is preferred to remove parenchymal cells and undesired tissue residues to a certain extent before applying the method of the present invention on them.

Parenchymal cells can be removed by a low speed centrifugation as described below. The low speed centrifugation is the condition which is sufficient to separate the light fraction which is enriched with nonparenchymal cells from the fraction mainly containing parenchymal cells and undesired tissue residues, preferably the condition sufficient to separate the light fraction which is enriched with small hepatocytes and nonparenchymal cells and which is substantially free of parenchymal cells from the fraction mainly containing parenchymal cells and undesired tissue residues. When the cells obtained from a liver according to the described method are fractionated under such conditions, the small hepatocytes may be obtained in the light fraction in larger quantities. Particularly, the cell suspension can be separated into a heavy fraction which mainly contains parenchymal cells and a light supernatant fraction which mainly contains relatively light cells including nonparenchymal cells such as stellate cells, Kupffer cells and sinusoidal endothelial cells by, for example, centrifuging at 50 x g for one (1) minute. Larger amount of the small hepatocytes may be obtained in the supernatant fraction under this centrifugation condition.

The centrifugation is preferably performed at about 50 x g for not more than 1 minute, because the ratio of precipitated small hepatocytes may increase as the speed or the time of centrifugation increases, although the ratio of parenchymal cells in the supernatant fraction decreases. Parenchymal cells and undesired tissue debris and the like may be further removed by repeatedly centrifuging, precipitating and suspending the supernatant fraction. More specifically, for example, the supernatant fraction may be centrifuged at 50 x g for 5 minutes and the precipitate is suspended in an appropriate medium and further centrifuged at 50 x g for 5 minutes. The precipitate is suspended in a similar medium, centrifuged again at 50 x g for 5 minutes. The precipitate is suspended in a similar medium and centrifuged at 150 x g for 5 minutes and the precipitated cells are suspended in a fresh medium. The number of cells in the cell suspension may be counted to adjust the cell density required for the subsequent culture or the treatments. Generally, the density is adjusted to 1 x 10⁴ to 5 x 10⁵ cells/ml.

Thus obtained fraction containing a large amount of small hepatocytes may be cultured or the small hepatocytes may be efficiently isolated from such prepared fraction according to the method of the present invention. The fraction which is prepared as described above and contains a large amount of small hepatocytes is preferably cultured in the presence of hyaluronic acid for 3 or more days, preferably 5 or more days to induce cell surface receptors of which ligand is hyaluronic acid before isolating the small hepatocytes. During the early culture the cells are preferably cultured in a serum-free medium for the first 24 hours after starting the culture to avoid non-specific adhesion of the cells. The expression of CD44, which is a receptor for hyaluronic acid, is likely to be distinguishing from about day 2 to day 3 of culture and reached to nearly a maximum at day 3 to day 5 of the culture as determined by RT-PCR. On the other hand, it has been known that CD44 is not expressed on mature hepatocytes (Terpe H-J, Stark H, Prehm P, Guenthert U., Histochemistry, 101, 79-89 (1994); Goodison S, Urquidi V, Tarin D., J. Clin. Pathol: Mol Pathol., 52, 189-196 (1999)).

The inventors contemplated the possibility that it would take 2 to 3 days for activating the small hepatocytes which have potentially existed, because the cell may be damaged by preparing the cell fraction from a liver. Additionally, the inventors confirmed that, when a rat received a severe damage by galactosamine or the like, CD44-positive cells appeared in the liver tissue after a few days of administrating galactosamine or the like, which is consistent with the aforementioned contemplation. Therefore, when the cell fraction is prepared from a liver of an individual which has received a severe damage by galactosamine or the like, small hepatocytes may be very efficiently isolated by a culture of shorter period in the presence of hyaluronic acid.

Alternately, the fraction which is prepared as described above and which contains a large amount of the small hepatocytes may be cultured for a period in the absence of hyaluronic acid (pre-culture) to proliferate the small hepatocytes before the small hepatocytes are efficiently cultured or isolated according to the present invention. Additionally, a cell fraction that can be obtained without enrichment as described about, or mature hepatocytes undergoing culture may be transferred to a culture in the presence of hyaluronic acid to culture and isolate the small hepatocytes. These cell fractions necessarily contain the small hepatocytes and the small hepatocytes are considered to occur during the period of culturing mature hepatocytes. Media and conditions for culturing normal human mature hepatocytes are described by the inventors in WO 02/24875 and normal human mature hepatocytes which has been cultured by using such media and conditions may be cultured in the presence of hyaluronic acid according to the present invention and may be subjected to separation.

When pre-culture is conducted, the fraction (cell population) which is prepared as described above and is enriched with small hepatocytes may be cultured in a basal medium containing serum, nicotinamide, vitamin C, antibiotics, growth factors and other additives commonly used for cell culture, for example Dulbecco's modified Eagle's medium supplemented with these substances, at 37°C. The medium for proliferating or maintaining the small hepatocytes preferably contains nicotinamide, vitamin C, growth factors, DMSO and the like. Vitamin C is usually added as ascorbic acid 2-phosphate preferably at a concentration of 0.1 mM to 1.0 mM, more preferably 0.5 to 1.0 mM, and nicotinamide is used at a relative high concentration, preferably 1 to 20 mM and more preferably 5 to 10 mM. As the growth factors, epidermal growth factor (EGF), hepatocyte growth factor (HGF), or transforming growth factor α (TGF α) and the like may be used. TGF α is particularly preferred. When TGF α is added, it is preferably to use it at a concentration of 1 µg/l to 100 µg/l, more preferably 5 µg/l to 50 µg/l. In the primary culture, DMSO is preferably added at a concentration of about 0.1 to about 2 % (v/v), more preferably about 0.5 % to about 1.5% (v/v) on and after day 4 after starting the culture.

For the container for the pre-culture, a culture container on which hyaluronic acid is attached such as those described below, but a culture dish conventionally used for cell culture may be also used. Generally, collagen-coated dishes are used for the culture of adherent cells. For example, the culture dishes of various sizes which are coated by collagen derived from bovine dermis or rat tail are commercially available. It is also possible to prepare such dishes, if necessary, but it is preferable to use dishes which are not coated by collagen because the cells can be dissociated from the dishes in the mild condition more easily when the extracellular matrix is less. In the isolation method or culture method according to the present invention, the pre-cultured cells may be disaggregated into individual cells in the presence of metal chelators and/or enzymes, but since the damages to the cells may be extensive, it is preferable to dissociate the colonies from culture containers by non-enzymatic procedures. The metal chelators which may be used are any ones as long as their cytotoxicity is less, for example EDTA, EGTA and/or the salts thereof may be used at the concentration generally used for them. Among non-enzymatic dissociation agents, a solution of Hank's buffer (pH 7.3 - 7.5) without Ca and Mg supplemented with EDTA, glycerol, sodium citrate and the like may be used. For example, a prepared non-enzymatic cell dissociation agent can be commercially available from Sigma Chemical Co., which is marketed as "Cell Dissociation Solution". For the culture, a conventional 5% CO₂ incubator may be used. The range of CO₂ concentration and the temperature are not essential as long as they are within the range which is acceptable for conventional cultured cells. Specifically, a cell suspension enriched with pre-cultured small hepatocytes can be obtained by using the methods and conditions described in WO 01/92481.

In one embodiment of the present invention hyaluronic acid is attached to a carrier (coating) and the small hepatocytes or cells from a liver of mammalian are co-cultured in the presence of the carrier on which hyaluronic acid has adhered. Any carrier may be used as long as it can be coated with hyaluronic acid and is suitable for cell culture. Such carriers include plastic products or glass products (e.g. culture dishes, culture flasks, culture bottles, plastic or glass beads), Sepharose, for example Sepharose beads, porous material (e.g. a sponge) coated with hyaluronic acid, metals and cellulose. When a cell culture container is used as the carrier, the container may be any one which is commonly used for culturing animal cells, but a carrier on which cells is unlikely to adhere inherently is preferable, such as uncoated cell culture containers or culture containers for bacteria.

In on embodiment of the present the small hepatocytes and/or cells from a liver of a mammalian are cultured in the presence of a carrier consisting of hyaluronic acid or a carrier of which the major component is hyaluronic acid, for example a porous material made of hyaluronic acid or a porous material (e.g. a sponge) of which the major component is hyaluronic acid. For this purpose, the culture container may be similar as in the cases of co-culture with a carrier coated with hyaluronic acid such as described above. A porous material is coated with hyaluronic acid and is preferable as a carrier because the area thereof for adhering the small hepatocytes is large. The porous material may be any one as long as it can be coated with hyaluronic acid and does not contain substances which are harmful to cells, especially to the small hepatocytes.

A carrier may be coated with hyaluronic acid, for example, as described below. Hyaluronic acid may be commercially available. In this connection, hyaluronic acid may be generally classified into low molecular weight hyaluronic acid and high molecular weight hyaluronic acid by setting a demarcation at molecular weight of about one million. The high molecular weight hyaluronic acid is preferably used in the present invention. Hyaluronic acid, preferably high molecular weight hyaluronic acid, may be prepared as a solution in an appropriate medium or buffer at a suitable concentration such that when 1 ml to 5 ml of the prepared solution is contacted with a carrier the amount of hyaluronic acid will be 10 µg/cm² to 1000 µg/cm² preferably 50 µg/cm² to 500 µg/cm² based on the surface area of the carrier. In general, the solution of hyaluronic acid will be conveniently prepared as a stock solution in an appropriate medium or buffer at a concentration of 10-20 mg/ml. The buffer used can be any buffer as long as it is not harmful for cells, including Hanks' solution, a traditional cell culture medium, saline, phosphate-buffered saline (PBS) or the like. The pH of the buffer may be within the range where hyaluronic acid can be dissolved, preferably 7.1 to 8.5. The temperature for dissolving hyaluronic acid may be any temperature as long as hyaluronic acid can be dissolved and does not decompose. It would be convenient to use 30°C to 40°C, preferably about 37°C, considering the temperature setting of a conventional incubator.

Thus prepared hyaluronic acid solution may be combined directly or optionally after diluting it with an appropriate buffer or medium together with a carrier such as described above to coat hyaluronic acid on the carrier. When a relatively water-repellent carrier is to be coated, it is particularly useful to increase the volume of the hyaluronic acid solution by dilution because the surface of the carrier may not sufficiently be covered by the hyaluronic acid solution if the volume of the solution for coating is small. For coating the carrier, hyaluronic acid solution of 0.5-20 mg/ml, preferably 1-10 mg/ml is contacted with the surface of the carrier at 10 µg to 1000 µg, preferably 50 µg to 500 µg per 1 cm² of the surface area of the carrier. For example, when a 60 mm-dish is to be coated with hyaluronic acid, a solution of hyaluronic acid prepared at a concentration of 10 mg/ml will be diluted to 1 mg/ml by PBS or the like and 1 ml of the dilute will be poured to the dish to coat it. The contact of the hyaluronic acid solution with the carrier is conducted by incubating it at 30°C to 40°C, particularly about 37°C for one to 24 hours. The temperature and period for incubation may be those sufficient for the hyaluronic acid to be coated on the carrier. The incubation period may be longer than 24 hours, but there would be less merit of long-term incubation.

Following a period which is sufficient for the coating, the hyaluronic acid solution is removed and the surface of the carrier is washed with a buffer which is not harmful to cells such as PBS. If appropriate, the carrier may be sterilized by, for example, UV irradiation. When the carrier itself is a culture container, an appropriate medium for the small hepatocytes such as a medium described below is placed in the container. When the carrier is a bead or a sponge or the like, it may be transferred to an appropriate culture container and the aforementioned medium may be added to the container. Cell suspensions which are expected to contain the small hepatocytes are placed into such prepared culture containers. The primary cell density for this purpose is preferably 1 x 10⁴ to 5 x 10⁵ cells/ml

The culture may be conducted in a basal medium containing serum, nicotinamide, vitamin C, antibiotics, growth factors and other additives commonly used for cell culture, for example Dulbecco's modified Eagle's medium supplemented with these substances, at 37°C in the presence of a hyaluronic acid-attached carrier. In one embodiment of the present invention, the hyaluronic acid-attached carrier may be a culture dish on which hyaluronic acid is applied or a culture flask on which hyaluronic acid is applied. In another embodiment, the hyaluronic acid-attached carrier may be a plastic bead, a glass bead or a Sepharose bead on which hyaluronic acid has attached or a magnetic bead on which hyaluronic acid has attached. In another embodiment the hyaluronic acid-attached carrier is a carrier made of hyaluronic acid or a carrier of which a major component is hyaluronic acid. Particularly a porous material (a sponge) is preferable as a carrier made of hyaluronic acid or a carrier of which major component is hyaluronic acid.

The medium preferably further contains nicotinamide, vitamin C, growth factors, DMSO and the like. Vitamin C is usually added as ascorbic acid 2-phosphate preferably at a concentration of 0.1 mM to 1.0 mM, more preferably 0.5 to 1.0 mM, and nicotinamide is used at a relative high concentration, preferably 1 to 20 mM and more preferably 5 to 10 mM. As the growth factors, epidermal growth factor (EGF), hepatocyte growth factor (HGF), or transforming growth factor α (TGF α) and the like may be used. TGF α is particularly preferred. When TGF α is added, it is preferably to use it at a concentration of 1 µg/l to 100 µg/l, more preferably 5 µg/l to 50 µg/l. When a cell fraction which has been prepared from a liver of mammalian and which contains the small hepatocytes is directly cultured (a primary culture) instead of culturing subcultured cells, DMSO is preferably added at a concentration of about 0.1 to about 2 % (v/v), more preferably about 0.5 % to about 1.5 % (v/v) on and after day 4 after starting the culture. Additionally, it is preferable to use a medium described above without supplementing serum for the first 24 hours and consequently the medium is preferably exchanged with the medium described above supplemented with serum. By culturing with a serum-free medium for the first 24 hours, specific adhesion by, for example, sinusoidal endothelial cells, may be reduced. It is also preferable to perform a primary culture with a serum-free medium to avoid vitronectin and fibronectin contamination.

A CO₂ incubator is used for the culture and the medium is preferably exchanged very other day (3 times a week) in general.

Specifically, the following medium may be used.

| Dulbecco's modified Eagle's medium (GIBCO Laboratories) | |
|---|---|
| + 20 mM HEPES | (Dojindo) |
| + 25 mM NaHCO₃ | (Katayama Chemical Co.) |
| + 30 mg/l proline | (Sigma Chemical Co.) |
| + 0.5 mg/l insulin | (Sigma Chemical Co.) |
| + 10⁻⁷ M dexamethasone | (Sigma Chemical Co.) |
| + 10% FBS | (Hyclone Laboratories, Inc.) |
| + 10 mM nicotinamide | (Katayama Chemical Co.) |
| + 1 mM L-ascorbic acid 2-phosphate | (Wako Pure Chemical Inc.) |
| + 10 µg/l EGF | (Collaborative Research Inc.) |
| + antibiotics | |
| 1 % dimethylsulfoxide (DMSO) | (ALDRICH) |

| | |
|---|---|
| (DMSO was added to the culture medium on and after day 4 of medium exchange.) | |

The period for the culture may be at least 2 or more days, preferably about 3 or more days, more preferably about 3 to 30 days, particularly preferably about 3 to 21 days when the cells are prepared from an individual which has not received a damage in the liver in advance. The small hepatocytes may be isolated in a short period when cells are prepared from an individual which has received a damage in the liver in advance or cells have been subcultured. For damaging a rat with galactosamine or the like, for example, an adult rat (8 to 10 weeks old) will receive D-galactosamine at a concentration of 10 mg to 100 mg of D-galactosamine (Across) in 200 µl PBS per 100g body weight by intraperitoneal injection. Cells may be isolated from the liver at day 3 to 6 post-injection according to the method of Seglen.

At the time of exchanging the medium, cells which do not attach to hyaluronic cells are easily removed from the culture, or cells which attach to hyaluronic acid may be easily recovered from the culture, because the cells which do not attach to hyaluronic cells are in a planktonic state or are likely to dissociate from the carrier. In the context of the present invention, removing cells which do not attach to hyaluronic acid from a culture is equivalent to recovering cells which attach to hyaluronic acid from a culture.

When the carrier to which hyaluronic acid has attached is a relatively large carrier such as a culture dish, a culture flask or a sponge, the cells which do not attach to hyaluronic acid may be easily removed by rinsing the surface of the carrier with an appropriate buffer or with a medium including PBS and the medium for culturing the small hepatocytes as described above. When the carrier is relatively small such as a bead, the cells which have attached to hyaluronic acid may be easily recovered or the cells which have not attached to hyaluronic acid may be easily removed by a centrifugation or by fixing the beads by magnetism in case of using magnetic beads, followed by removing the medium and optionally washing them with an appropriate buffer or a medium. Consequently, the cells may be further cultured in the presence or absence of hyaluronic acid after adding a fresh medium, or the culture may be subjected to a treatment for releasing the small hepatocytes from the carrier as described below.

Following the culture as described above, the medium is removed and optionally the carrier is washed with a medium or an appropriate buffer such as PBS, and the cells which attached to hyaluronic acid may be released from the carrier by acting hyaluronidase on the carrier. Hyaluronidase is commercially available and may be prepared and used in an appropriate buffer. The appropriate buffer used may be any buffer as long as it can dissolve a hyaluronidase and did not significantly inhibit an activity of the hyaluronidase. For example, Hanks' solution, a conventional cell culture medium, saline or phosphate buffered saline (PBS) may be used. The concentration of the hyaluronidase is not also particularly restricted, and the hyaluronidase may be prepared at a concentration of, for example, 0.1-20 mg/ml, preferably 1-10 mg/ml. For releasing small hepatocytes from a carrier, such prepared hyaluronidase may be acted on the carrier-hyaluronic acid-cells complex. The amount for hyaluronidase to be functioned is not particularly restricted, and the period for incubation may be adjusted depending on the amount used. However, about 5 µg to 1000 µg, preferably about 50 µg to 500 µg of hyaluronidase per 1 cm² of the surface area of the carrier may be used.

For example, after removing the medium and washing with PBS, 1 ml of 1 mM EDTA/PBS is added to the complex which is then incubated for one hour at room temperature. After removing 1 mM EDTA/PBS, hyaluronidase solution of the above described concentration, which has been pre-warmed at the same temperature as that for culture, for example 37°C, is added to the complex which is incubated for an appropriate time at 37°C in a 5% CO₂ incubator. For example, when a 60 mm-culture dish is used, about 1 ml of hyaluronidase solution at a concentration of 5 mg/ml or 10 mg/ml is preferably used. The incubation period may be typically about 30 minutes to about 2 hours, when a hyaluronic acid-attached carrier such as a culture dish or a flask coated with hyaluronic acid is used. Particularly a carrier made of hyaluronic acid or a hyaluronic acid-based carrier is treated, including a hyaluronic acid-sponge or the like, the incubation period is such that the entire carrier or the hyaluronic acid which is the major component of the carrier is sufficiently lysed. In either case, the temperature or the period of incubation may be appropriately adjusted by monitoring the release of the cells. Consequently, the small hepatocytes may be recovered by adding an appropriate volume of an ice-cold medium, mildly pipetting, transferring them into a tube followed by a centrifugation for 5 minutes at 50 x g.

Depending on the condition including the culture period and the like, the small hepatocytes may not be sufficiently released by a treatment with hyaluronidase. In such cases, a commercially available cell dissociation solution such as "Cell Dissociation Solution" which is available from Sigma may be used. For example, after removing the culture medium the carrier may be washed with an appropriate buffer such as PBS or the like, and to an appropriate volume of 1 mM EDTA/PBS is added to the carrier which is then incubated for 1 to 3 minutes at a room temperature. Then the small hepatocytes may be easily released from the carrier, by adding an appropriate volume of "Cell Dissociation Solution", which has been pre-warmed to the temperature for culturing (e.g. 37°C), to the carrier after removing 1 mM EDTA/PBS, and incubating the carrier, for example for 5 to 15 minutes, in a 5 % CO₂ incubator. The temperature or the period of incubation may be appropriately adjusted by monitoring the release of the cells.

The aforementioned treatment with a cell dissociation solution may be performed alone or in combination with a hyaluronidase treatment.

Following the incubation, the small hepatocytes released from the carrier may be recovered by using an appropriate method. For example, following the incubation, to the culture container an appropriate volume of a buffer or a medium which not harmful to the cells, including PBS, is added to prepare a cell suspension of small hepatocytes and the small hepatocytes released from the carrier-hyaluronic acid can be washed and recovered by a centrifugation and re-suspending the cells with optionally repeating the steps. The centrifugation is preferably conducted at a low speed for a short period, for example about 5 minutes at 50 x g, not to extremely damage the small hepatocytes. When the carrier is relatively small such as beads or the like, the small hepatocyte suspension may be separated from the carrier-hyaluronic acid by a centrifugation at a lower speed and/or for a shorter period or alternately by simply standing them for a few seconds to a few minutes, preferably one minute or less. Thus obtained small hepatocytes may be further cultured again in the presence of a carrier of which surface has been attached by hyaluronic acid and/or in the presence of a carrier of which a major component is hyaluronic acid, with repeating the steps described above.

Various markers may be used to confirm that such isolated small hepatocytes have some of the functions of hepatocytes. Such markers include, besides albumin which is secreted in the medium, transferrin, a production of urea, an amount of glycogen, cytochrome P450 and the like. These markers may be confirmed by ELISA, Western blot analysis, or RT-PCR and the like for the medium or the cell extract, or by directly immunostaining the cells. The small hepatocytes can be distinguished from other cells in that they exhibit the markers of mature hepatocytes such as albumin, CK8, CK18, glycogen or peroxisome but they do not exhibit the markers of biliary epithelial cells such as CK7 or CK19. For example, oval cell, which is known to be a kind of hepatic stem cells, exhibits the markers of biliary epithelial cells such as CK7 and CK19 but it does not exhibit the markers of mature hepatocytes such as glycogen or peroxisome. Additionally, it can be confirmed that other markers for non-parenchymal cells (ED1/2 (a marker for Kupffer cells), SE-1 (a marker for sinusoidal endothelial cells), desmin (a marker for Ito cells) and vimentin (a marker for liver epithelial cells)) are negative.

The efficiently isolated small hepatocytes as such may be cultured in analogy to the culture of the fraction which is obtained from a liver and contains a large amount of small hepatocytes by using the medium and culture container as described above. For this purpose, a hyaluronic acid-attached container may be used as described above as a culture container, although a culture dish which is conventionally used for cell culture may be also used. A collagen-coated dish which is conventionally used for adherent cells may be used, but is preferable to use a culture dish which is not coated with collagen when the small hepatocytes are to be dissociated from the culture container such as in the preparation of colonies for cryopreservation of colonies suitable for forming a liver tissue, because the cells can be dissociated from the dishes in the mild condition more easily when the extracellular matrix is less and the small hepatocytes are likely to be preferentially dissociated from the dishes. A conventional 5% CO₂ incubator may be used for the culture. The range of CO₂ concentration and the temperature are not essential as long as they are within the range which is acceptable for conventional cultured cells.

Additionally, such efficiently isolated, cultured and maintained small hepatocytes can be cryopreserved according to the method described in WO 01/092481 or matured to a liver tissue according to the method described in WO 02/088332, and are also used to estimate a drug function by using the matured liver tissue.

The disclosure of the references cited herein is entirely incorporated by reference.

### Example

### Example 1. Preparation of a fraction enriched with small hepatocytes

Livers of adult rats (10 to 15 weeks old) were perfused through portal vein by Ca, Mg-free Hank's solution supplemented with 0.2 mM EGTA according to the similar method of Seglen. Hank's solution was perfused for about 4 minutes at a flow rate of 40 ml/min, and then Hank's solution containing 0.02% collagenase (Yakult Co.) was perfused for 10 minutes at a flow rate of 20 ml/min. The hepatocytes were dropped into a beaker from the digested liver according to an ordinary method. The cell suspension was passed through a 250 µm- and then 70 µm-mesh filter and centrifuged for one minute at 50 x g. The supernatant was collected and centrifuged twice for one minute at 50 x g. The supernatant was collected and centrifuged for 5 minutes at 50 x g. The precipitated cells were washed with a medium (Leibovitz L-15 + 10% fetal bovine serum + 10⁻⁷ M dexamethasone + 0.5 mg/ml insulin + antibiotics) and were centrifuged for 5 minutes at 50 x g. The similar procedure was repeated and the suspension was centrifuged again for 5 minutes at 150 x g. The similar procedure was repeated and the suspension was centrifuged again for 5 minutes at 50 x g. The precipitated cells were suspended in the fresh medium and the number of viable cells was counted to adjust the cell density to 1.0 x 10⁴ to 5 x 10⁵ cells/ml.

### Example 2. Analysis of expression pattern of CD44 on small hepatocytes

### 1) Time course of expression pattern of CD44 BRI3

Total RNA was prepared from small hepatocytes immediately after preparation or after starting culture, and the expression of CD44 was determined by RT-PCR method. GPDH (glycerol-3-phosphate dehydrogenase) was used as a positive control (a housekeeping gene). The following medium was used for the culture:

| Dulbecco's, modified Eagle's medium (GIBCO Laboratories) | |
|---|---|
| + 20 mM HEPES | (Dojindo) |
| + 25 mM NaHCO₃ | (Katayama Chemical Co.) |
| + 30 mg/l proline | (Sigma Chemical Co.) |
| + 0.5 mg/l insulin | (Sigma Chemical Co.) |
| + 10⁻⁷ M dexamethasone | (Sigma Chemical Co.) |
| + 10% FBS | (Hyclone Laboratories, Inc.) |
| + 10 mM nicotinamide | (Katayama Chemical Co.) |
| + 1 mM L-ascorbic acid 2-phosphate | (Wako Pure Chemical Inc.) |
| + 10 µg/l EGF | (Collaborative Research Inc.) |
| + antibiotics | |
| 1 % dimethylsulfoxide (DMSO) | (Aldrich) |

| | |
|---|---|
| (DMSO was added to the culture medium on and after day 4 of medium exchange.) | |

Total RNA was prepared from about 1.0-1.5 x 10⁵ small hepatocytes by using ISOGEN (NIPPON GENE Co.). The cells were lysed according to the protocol recommended by the manufacturer, chloroform was added to the lysate and mixed well, and then the lysate was centrifuged at 4°C for 15 minutes at 10000 x g. The upper layer was removed and isopropanol was added to it, and centrifuged at 4°C for 15 minutes at 10000 x g, and the resulting precipitate was washed with 70% ethanol and dissolved in DEPC-water. RNA from mature hepatocytes was also extracted from cells immediately after perfusion by using the similar method.

Ten nanogram of the resulting total RNA was used for RT-PCR. The PCR product was subjected to electrophoresis and each band was detected by a densitometer. The intensity of CD44 band was divided by the intensity of GPDH band to calculate the relative intensity. The primer used was as follows.
5'-cccgaattcatggacaaggtttggtggca-3' (SEQ ID NO:1)
5'-cccgaattcctacaccccaatcttcatat-3' (SEQ ID NO:2)

The following primers were used for GPDH:
5'-accacagtccatgccatcac-3' (SEQ ID NO:3)
5'-tccaccaccctgttgctgta-3' (SEQ ID NO:4)
The condition for PCR was as follows : 95°C-15 sec., 60°C-30 sec., 68°C-1 min., cycles=25

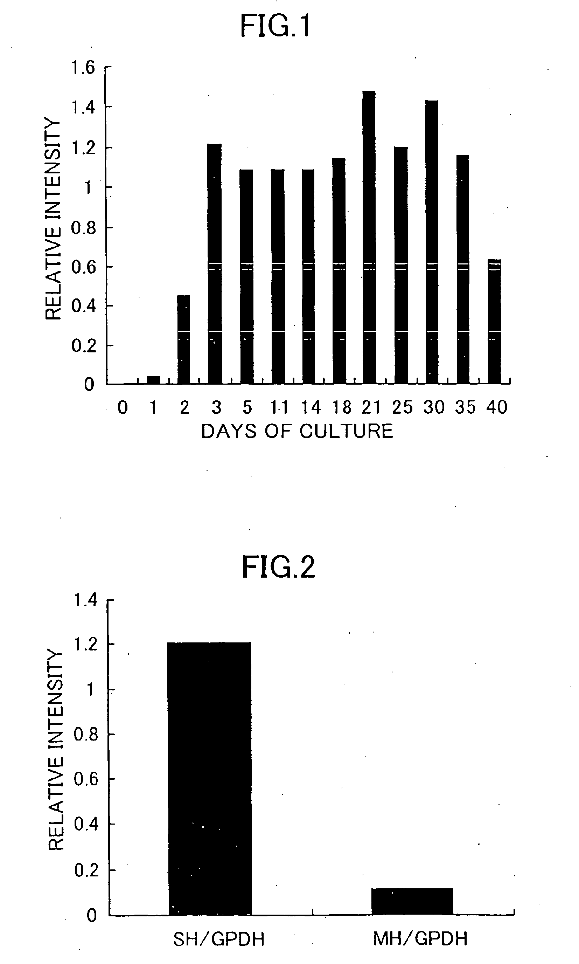

The results revealed that the significant expression of CD44 was observed at about 2 to 3 days after starting the primary culture and that the expression continued to increase up to about a maximum 5 days after starting the culture and the expression level of CD44 was maintained in the proliferating small hepatocytes (Figure 1). It was also shown that the expression of CD44 reduced at day 44 of the culture where the small hepatocytes matured on some level (Figure 1).

### 2) Comparison of the expression patterns of CD44 in small hepatocytes and in mature hepatocytes

The cells (about 1.0-1.5 x 10⁸ cells) which had been cryopreserved according to the method described in WO 01/92481 were thawed according to a conventional procedure and cultured for two weeks. Total RNA was extracted from the cultured cells by using the similar procedure described in 1). RNA from mature hepatocytes was extracted from the cells immediately after the perfusion by using the similar method. Ten nanogram of the obtained total RNA was used for determining the relative expression level of CD44 by using RT-PCR similarly as described in 1), except that the number of cycle was 35. The PCR product was subjected to electrophoresis and each band was detected by a densitometer, and the intensity of CD44 band was divided by the intensity of GPDH band to compare the expression of CD44 in small hepatocytes and that in mature hepatocytes as relative intensities (Figure 2).

The results revealed that the expression of CD44 was consistently and extremely low in the mature hepatocytes (Figures 1 and 2)

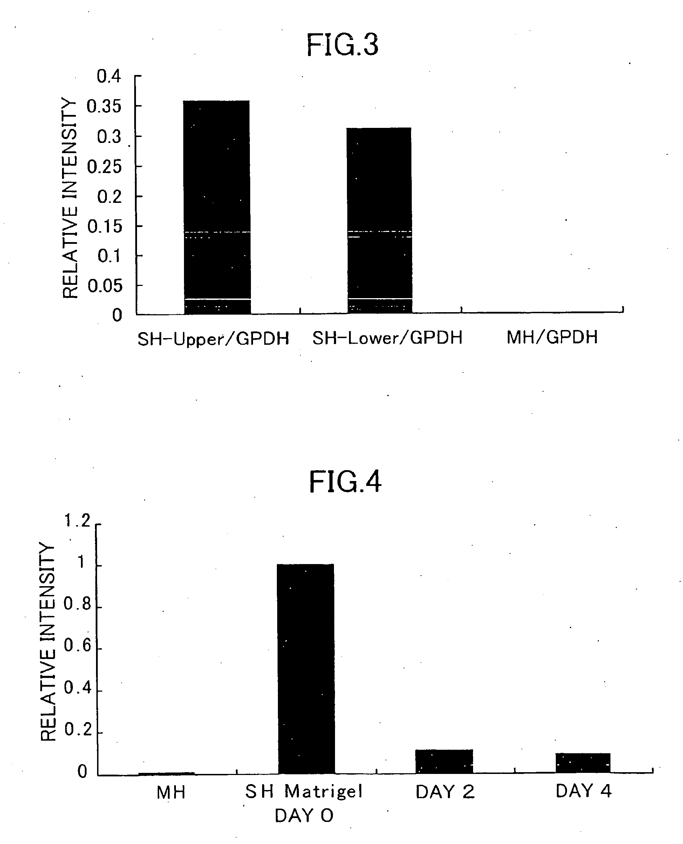

Similar results were obtained with Northern blot (Figure 3). Again, in this case GPDH was used as a housekeeping gene. The resulting bands were read out by a densitometer as described above, and the intensity of CD44 band was divided by the intensity of GPDH band to compare the expression of CD44 in small hepatocytes and in mature hepatocytes. The probe used was the full length of the coding region of CD44 (SEQ ID NO:5). The probe was labeled by using Alkphos Direct Labeling Kit (Amersham Biosciences).

Additionally, the change in CD44 expression was determined by Northern blot after inducing the maturation of small hepatocytes by adding Matrigel into the cultured small hepatocytes according to the method described in WO 02/088332. It has been known that the maturation started at day 2 after adding Matrigel. It was shown that the expression of CD44 decreased concurrently with the maturation (Figure 4).

These results demonstrate that CD44 is specific to small hepatocytes and also suggest the possibility that the expression of CD44 is induced by a damage of preparing a cell fraction form a liver and that small hepatocytes which have potentially existed in the cell population composing a liver or precursors thereof elicited their inherent properties.

### Example 3. Culture in the presence of hyaluronic acid

A stock solution of hyaluronic acid was prepared in PBS to the concentration of 10 mg/ml, and kept at 37°C. The hyaluronic acid used was purchased from Sigma (Sigma Hyaluronic acid (human umbilical cords), Catalog No. H-1504). To the resulting hyaluronic acid stock solution, an appropriate volume of PBS was added to prepare a diluted solution of hyaluronic acid such that one to 10 mg of hyaluronic acid is contained in a total volume of 3 ml. The resulting hyaluronic acid solution at different concentrations was added to an untreated 60 mm-dish (Kord-Valmark Co.). The dish was incubated at 37°C overnight and washed with PBS before use.

The cell fraction enriched with small hepatocytes, which had been prepared as described in 1), was plated on the dish. The following medium was used for the first 24 hours, and the medium was exchanged to the aforementioned medium supplemented with 10% FBS (Hyclone Laboratories, Inc.). A CO₂ incubator was used for the culture and the medium was exchanged very other day (3 times a week) in general.

The medium composition is as follows:

| Dulbecco's Modified Eagle Medium (GIBCO Laboratories) | |
|---|---|
| + 20 mM HEPES | (Dojindo) |
| + 25 mM NaHCO₃ | (Katayama Chemical Co.) |
| + 30 mg/l proline | (Sigma Chemical Co.) |
| + 0.5 mg/l insulin | (Sigma Chemical Co.) |
| + 10⁻⁷ M dexamethasone | (Sigma Chemical Co.) |
| + 10 mM nicotinamide | (Katayama Chemical Co.,) |
| + 1 mM L-ascorbic acid 2-phosphate | (Wako Pure Chemical Ind.) |
| + 10 µg/l EGF | (Collaborative Research Inc.) |
| + antibiotics | |
| dimethylsulfoxide (DMSO) | (ALDRICH) |

| | |
|---|---|
| (DMSO was added to the culture medium on and after day 4 of medium exchange) | |

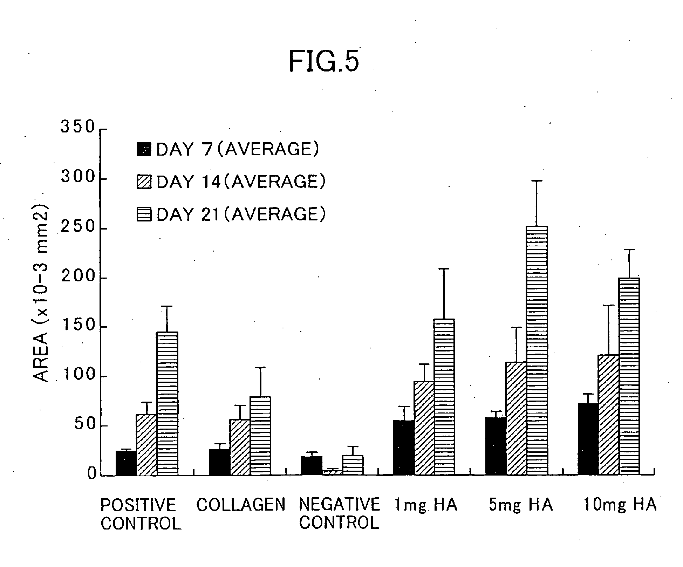

Culture dishes of 60-mm were coated with hyaluronic acid by using 1 mg, 5 mg or 10 mg of hyaluronic acid and the proliferation of the small hepatocytes on the culture dishes coated with hyaluronic acid was monitored at day 7, 14 and 21 with a microscope. The results were shown in Figure 5.

As can be apparent from Figure 5, the small hepatocytes could hardly form colonies on a culture dish for non-adherent cells (negative control), and the area of the small hepatocytes were larger when the culture is conducted on the dishes coated with hyaluronic acid as compared with the cell area cultured on a culture dish for adherent cell (positive control). Additionally, it was shown that when the culture period increased, the small hepatocytes would differentiate (i.e. a maturation would occur) instead of proliferating and the increase rate in the areas of the colony of the small hepatocytes declined, but the area was still larger at least until day 21 as compared to the case where hyaluronic acid was not applied. Any morphological change was not detected as observed by a microscope.

### Example 4. Isolation of small hepatocytes

### a) Treatment with hyaluronidase

The small hepatocytes were cultured for 6 days or 3 weeks on a culture dish on which hyaluronic acid was applied before treating them with hyaluronidase. The medium was removed from the dishes and the dishes were washed with PBS. To the dishes 1 ml of 1 mM EDTA/PBS was added and the dishes were incubated for one minute at room temperature. 1m M EDTA/PBS was removed and 1 ml of hyaluronidase (5 mg/ml or 10 mg/ml) which had been pre-warmed at 37°C was added to the dishes, which was further incubated for 2 hours at 37°C in a 5% CO₂ incubator. Consequently 1ml of ice-cold medium was added, mildly pipetted, and the cells were harvested in a tube which was centrifuged for 5 minutes at 50 x g to recover the small hepatocytes.

The colonies of small hepatocytes which had been cultured for 6 days were able to be detached and recovered, but cells which had been cultured for 3 weeks could not be fully detached by using a hyaluronidase alone. Therefore, a dissociation solution was used as described in b).

### b) Treatment with Cell Dissociation Solution

After culturing the cells for 3 weeks on a culture dish on which hyaluronic acid was applied, the cells adhered on the culture dish were treated with Cell Dissociation Solution (Sigma Chemical Co.) according to the protocol recommended by the manufacturer's.

The medium was removed from the dishes and the dishes were washed with PBS. To the dishes 1 ml of 1 mM EDTA/PBS was added and incubated for one minute at room temperature. After removing 1 mM EDTA/PBS, 1 ml of Cell Dissociation Solution which had been pre-warmed at 37°C was added to the dishes which were consequently incubated for 5-15 minutes at 37°C in a 5% CO₂ incubator. Then 1 ml of ice-cold medium was added, and the medium was collected into a tube which was then centrifuged to recover the small hepatocytes.

The obtained cells were morphologically comparable with small hepatocytes and were CD44-positive. The cells could proliferate to form colonies. The cells which formed the colonies secreted albumin and were negative for markers for non-parenchymal cells (ED1/2 (a marker for Kupffer cells), SE-1 (a marker for sinusoidal endothelial cells), desmin (a marker for Ito cells) and vimentin (a marker for liver epithelial cells)) as analyzed by immunostaining. Thus, the obtained cells were confirmed to be the small hepatocytes.

### Example 5. Preparation of mature hepatocytes

A liver of an adult rat was perfused with a solution containing collagenase. The resulting cell suspension was centrifuged for 1 minute at 4°C and 50 x g to recover a precipitate. The precipitate was suspended in Hank's buffer and centrifuged for 1 minute at 4°C and 50 x g, which was repeated three times.

The resulting cells were suspended in 25 ml of Hank's buffer. The suspension was added to 21.6 ml of Percoll and 2.4 ml of 10x Hank's buffer, and mixed by inversion. The supernatant was removed after centrifugation for 15 minutes at 4°C and 50 x g. The cells were suspended in PBS, centrifuged for 1 minute at 4°C and 50 x g and the precipitate was recovered to obtain mature hepatocytes.

### Example 6. Comparison of the cultures of small hepatocytes and mature hepatocytes in the presence of hyaluronic acid

The small hepatocytes and mature hepatocytes, which had been prepared according to the method described in Examples 1 and 4, was plated on dishes coated according to the method described in Example 3. The culture was conducted similarly as described in Example 3.

The following medium was used for the first 24 hours and the medium was then replaced with the same medium supplemented with 10% FBS (Hyclone Laboratories, Inc.). A CO₂ incubator was used for the culture, and the medium was change 3 times a week.

| Dulbecco's Modified Eagle Medium (GIBCO Laboratories) | |
|---|---|
| + 20 mM HERPES | (Dojindo) |
| + 25 mM NaHCO₃ | (Katayama Chemical Co.) |
| + 30 mg/l proline | (Sigma Chemical Co.) |
| + 0.5 mg/l insulin | (Sigma Chemical Co.) |
| + 10⁻⁷ M dexamethasone | (Sigma Chemical Co.) |
| +10 mM nicotinamide | (Katayama Chemical Co.,) |
| + 1 mM L-ascorbic acid 2-phosphate | (Wako Pure Chemical Ind.) |
| + 10 µg/l EGF | (Collaborative Research Inc.) |
| + antibiotics | |
| 1 % dimethylsulfoxide (DMSO) | (ALDRICH) |

| | |
|---|---|
| (DMSO was added to the culture medium on and after day 4 of medium exchange.) | |

Numbers of the cells grown on the dishes were counted at day 1 and day 7 of the culture. The ratio of the number of cells at day 7 to that of day 1 was indicated designating the number of cells at day 1 as one (Figure 6). The mature hepatocytes hardly grew at day 7 of culture, while the number of the small hepatocytes reached to about 7-fold at day 7 as compared to the number of cells at day 1.

According to the present invention, small hepatocytes can be efficiently isolated from a liver of a mammalian including human being. According to the present invention, the small hepatocytes can be selectively cultured. Furthermore, the small hepatocytes can be efficiently separated and cultured distinguishing them from cells which have been matured during the culture and lost the permanent proliferative capacity.

For example, when the small hepatocytes and mature hepatocytes were cultured respectively on dishes coated with hyaluronic acid, the mature hepatocytes hardly grew at day 7 of the culture while the small hepatocytes reached to the number of cells about 7-fold higher at day 7 as compared to the number of cells at day 1, which leads to the possibility that the small hepatocytes can be isolated and separately cultured from mature hepatocytes.

### References

1. International Publication WO 01/92481
2. International Publication WO 02/088332
3. Japanese Patent No. 3211941
4. Laid Open Publication of Japanese Patent Application (JP-Kokai) No. 2002-078481
5. JP-Kokai No.09-313172
6. JP-Kokai No.08-112092
7. JP-Kokai No. 2002-045087
8. Mitaka T. et al., Hepatology 16, 440-447 (1992)
9. Mitaka T., Sato F., Mizuguchi T. et al., Hepatology 29, 111-135 (1999)

## Claims

1. A method of isolating small hepatocytes, comprising the steps of
i) culturing cells from a liver of mammalian in the presence of a carrier of which surface is attached by hyaluronic acid and/or in the presence of a carrier of which a major component is hyaluronic acid; and,
ii) recovering cells which adhere to hyaluronic acid from the culture obtained in step i).

2. The method of isolating small hepatocytes according to claim 1, comprising the steps of
i) culturing cells from a mammalian liver in the presence of a carrier of which surface is attached by hyaluronic acid and/or in the presence of a carrier of which a major component is hyaluronic acid;
ii) in the culture obtained in step i) separating cells which adhere to hyaluronic acid from cells which do not adhere to hyaluronic acid;
iii) releasing the cells which are obtained in step ii) and which adhere to hyaluronic acid from the hyaluronic acid;
iv) repeating the steps i) to iii) once or more for the small hepatocytes obtained in step iii); and
v) recovering the cells released from hyaluronic acid.

3. The method of isolating small hepatocytes according to claim 1 or 2, wherein the carrier is selected from the group consisting of porous materials, glasses, Sepharoses, plastics, metals and cellulose.

4. The method of isolating small hepatocytes according to any one of claims I to , wherein the surface of the carrier is attached by incubating the carrier with hyaluronic acid at 10µg to 1000µg per 1 cm² of the surface area of the carrier.

5. The method of isolating small hepatocytes according to any one of claims 1 to 4, wherein the step of releasing the small hepatocytes from hyaluronic acid is conducted by an act of hyaluronidase.

## Patentansprüche

1. Verfahren zum Isolieren kleiner Hepatocyten, umfassend die Schritte
(i) Kultivieren von Leberzellen eines Säugetieres in Gegenwart eines Trägers mit durch Hyaluronsäure befestigter Oberfläche und/oder in Gegenwart eines Trägers mit einem Hauptbestandteil an Hyaluronsäure, und
(ii) Gewinnen von Zellen aus der Kultur erhalten in Schritt (i), die an Hyaluronsäure anhaften.

2. Verfahren zum Isolieren kleiner Hepatocyten gemäß Anspruch 1, umfassend die Schritte
(i) Kultivieren von Leberzellen eines Säugetieres in Gegenwart eines Trägers mit durch Hyaluronsäure befestigter Oberfläche und/oder in Gegenwart eines Trägers mit einem Hauptbestandteil an Hyaluronsäure, und
(ii) Gewinnen von Zellen aus der Kultur erhalten in Schritt (i), die an Hyaluronsäure anhaften;
(iii) Freisetzen der in Schritt (ii) erhaltenen Zellen, die an Hyaluronsäure binden, von der Hyaluronsäure;
(iv)ein- oder mehrmaliges Wiederholen der Schritte (i) bis (iii) mit den in Schritt (iii) erhaltenen kleinen Hepatocyten, und
(v) Gewinnen der von Hyaluronsäure freigesetzten Zellen.

3. Verfahren zum Isolieren kleiner Hepatocyten gemäß Anspruch 1 oder 2, wobei der Träger ausgewählt ist aus der Gruppe bestehend aus porösen Materialien, Gläsern, Sepharosen, Plastik, Metallen und Zellulose.

4. Verfahren zum Isolieren kleiner Hepatocyten gemäß einem der Ansprüche 1 bis 3, wobei die Oberfläche des Trägers angeheftet ist durch Inkubieren des Trägers mit Hyaluronsäure bei 10 bis 1000µg pro cm² an die Träger-Oberfläche.

5. Verfahren zum Isolieren kleiner Hepatocyten gemäß einem der Ansprüche 1 bis 4, wobei der Schritt des Freisetzens kleiner Hepatocyten von Hyaluronsäure ausgeführt wird durch eine Einwirkung von Hyaluronidase.

## Revendications

1. Procédé d'isolement de petits hépatocytes, comprenant les étapes de
i) culture de cellules provenant d'un foie de mammifère en présence d'un support dont une surface est liée par l'acide hyaluronique et/ou en présence d'un support dont un composant majeur est l'acide hyaluronique ; et
ii) récupération des cellules qui adhèrent à l'acide hyaluronique à partir de la culture obtenue dans l'étape i).

2. Procédé d'isolement de petits hépatocytes selon la revendication 1, comprenant les étapes de
i) culture de cellules provenant d'un foie de mammifère en présence d'un support dont une surface est liée par l'acide hyaluronique et/ou en présence d'un support dont un composant majeur est l'acide hyaluronique ;
ii) dans la culture obtenue dans l'étape i), séparation des cellules qui adhèrent à l'acide hyaluronique des cellules qui n'adhèrent pas à l'acide hyaluronique ;
iii) libération des cellules qui sont obtenues dans l'étape ii) et qui adhèrent à l'acide hyaluronique de l'acide hyaluronique ;
iv) répétition des étapes i) à iii) une ou plusieurs fois pour les petits hépatocytes obtenus dans l'étape iii) ; et
v) récupération des cellules libérées de l'acide hyaluronique.

3. Procédé d'isolement de petits hépatocytes selon la revendication 1 ou 2 où le support est choisi dans le groupe consistant en les matériaux poreux, les verres, les Sepharoses, les matières plastiques, les métaux et la cellulose.

4. Procédé d'isolement de petits hepatocytes selon l'une quelconque des revendications 1 à où la surface du support est liée par incubation du support avec de l'acide hyaluronique à raison de 10 µg à 1 000 µg par cm² d'aire du support.

5. Procédé d'isolement de petits hépatocytes selon l'une quelconque des revendications 1 à 4 où l'étape de libération des petits hépatocytes de l'acide hyaluronique est conduite par une action de hyaluronidase.


### SEQUENCE LISTING

<110> Japan Science and Technology Agency
<120> Selective culture method and isolation method of small I hepatocytes with hialuronic acid
<130> Y1M0397
<150> JP 2004-191477
   <151> 2004-06-29
<160> 5
<170> Patent In version 3.2
<210> 1
   <211> 29
   <212> DNA
   <213> Artificial
<220>
   <223> PCR primer
<400> 1
   cccgaattca tggacaaggt ttggtggca 29
<210> 2
   <211> 29
   <212> DNA
   <213> Artificial
<220>
   <223> PCR primer
<400> 2
   cccgaattcc tacaccccaa tcttcatat 29
<210> 3
   <211> 20
   <212> DNA
   <213> Artificial
<220>
   <223> PCR primer
<400> 3
   accacagtcc atgccatcac 20
<210> 4
   <211> 20
   <212> DNA
   <213> Artificial
<220>
   <223> PCR primer
<400> 4
   tccaccaccc tgttgctgta 20
<210> 5
   <211> 1095
   <212> DNA
   <213> Rattus norvegicus
<400> 5
   atggacaagg tttggtggca cacagcttgg ggactacttt gcctcttaca gttgagcctg     60
   gcacagcagc agatcgattt gaatataacc tgccgttacg caggtgtatt ccatgtggag    120
   aaaaatggcc gctacagtat ctccaggact gaagcagctg acctctgcga ggctttcaac    180
   accaccttgc ccaccatggc tcagatggag ttagccctga gaaaggggtt tgaaacatgc    240
   aggtatgggt tcatagaagg acacgtggta atcccgagga tccaccccaa cgctatctgt    300
   gcagccaaca acacaggagt gtatatcctc ctcgcatcca acacctccca ctatgacaca    360
   tattgcttca atgcctcagc tcctcttgaa gaagactgta catcagtcac agacctaccc    420
   aattccttcg atggaccagt taccataact attgtcaacc gtgatggcac ccgctacagc    480
   aagaagggcg agtatagaac acaccaagaa gacatcgatg cctcaaacat tatagatgag    540
   gatgtcagca gtggatccac cattgagaag agcaccccag aaggctacat tttgcacacc    600
   gaccttccca cttcacagcc tactggagac cgggatgacg ccttctttat tgggagcacc    660
   ctggccacca gtgatggaga ctcatccatg gaccccaggg gtggtttcga cactgtgact    720
   catggatccg aattagctgg acactcaagt gggaatcaag acagtggagt gaccacaact    780
   tctggtcctg cgaggagacc tcagattcca gagtggctta tcatcttggc atccctcctg    840
   gcgctggctc tgattcttgc cgtctgcatt gctgtcaaca gtaggagaag gtgtgggcag    900
   aagaagaagc tggtgatcaa cagtggcaat ggaacagtgg aagacaggaa accaagtgaa    960
   ctcaacgggg aggccagcaa gtctcaggaa atggtgcatt tggtgaacaa ggaaccaaca   1020
   gagactccgg accagtttat gacagctgat gagacccgga atctgcagag tgtggatatg   1080
   aagattgggg tgtag                                                    1095